# FASCICULE DE BREVET EUROPEEN

(11) **EP 0 564 643 B1**
(45) Date de publication et mention de la délivrance du brevet: **01.05.1996**
(21) Numéro de dépôt: 92923850.9
(22) Date de dépôt: 21.10.1992
(51) Int. Cl.: C07C 217/08, C09D 5/44, C07C 271/20, C07C 271/28

(54) **PRODUITS DE REACTION D'AMINE-DIOL ET DE SUBSTANCE POLYFONCTIONNELLE ET LEUR APPLICATION DANS LES COMPOSITIONS DE PEINTURES CATIONIQUES ELECTRODEPOSABLES**
REAKTIONPRODUKTEN VON EINEM AMINDIOL MIT EINEM POLYFUNKTIONELLEN VERBINDUNG UND IHRE VERWENDUNG IN CATIONISCHER LACKIERUNGZUSAMMENSETZUNGEN
PRODUCTS OBTAINED FROM THE REACTION OF AMINE-DIOL AND A POLYFUNCTIONAL SUBSTANCE, AND APPLICATION OF SUCH PRODUCTS TO ELECTROAPPLICABLE CATIONIC PAINT COMPOSITIONS

(30) Priorité: 23.10.1991 FR 9113118
(43) Date de publication de la demande: 13.10.1993
(73) Titulaire: PPG INDUSTRIES (FRANCE) S.A., F-59307 Valenciennes Cédex (FR)
(72) Inventeur: LE DISERT, Yves, F-93700 Drancy (FR); ROUE, Jean, F-77600 Bussy-S.-Georges (FR); MORIARITY, Thomas, Wexford, PA 15090 (US); FAUCHER, Philippe, F-75019 Paris (FR)
(74) Mandataire: Phélip, Bruno
(86) Numéro de dépôt international: FR9200988
(87) Numéro de publication internationale: WO9308157

(56) Documents cités:
- EP-A- 0 011 130
- EP-A- 0 193 685
- GB-A- 2 187 449
- US-A- 4 001 329
- US-A- 4 588 840
- US-A- 4 761 502
- US-A- 4 810 535
- CHEMICAL ABSTRACTS, vol. 101, no. 24, 10 Décembre 1984, Columbus, Ohio, US; abstract no. 215560x, D. BRODRECHT ET. AL. 'Cationic emulsion for road construction' page 364 ;colonne 2 ; & DD,A,207 811
- CHEMICAL ABSTRACTS, vol. 104, no. 21, 26 Mai 1986, Columbus, Ohio, US; abstract no. 185507c, B. KEIL ET. AL. 'Soil Stabilisers' page 561 ;colonne 1 ; & DD,A,228 417
- CHEMICAL ABSTRACTS, vol. 97, no. 22, 29 Novembre 1982, Columbus, Ohio, US; abstract no. 184144g, CANON, K.K. 'Jet Printing Inks' page 102 ;colonne 1 ; & JP,A,82 090 065
- CHEMICAL ABSTRACTS, vol. 79, no. 25, 24 Décembre 1973, Columbus, Ohio, US; abstract no. 151696p, S MAEDA 'Desensitiver compositions for pressure sensitive copying paper' page 364 ;colonne 2 ; & ZA,A,7 203 908

## Description

La présente invention concerne le domaine des peintures électrodéposables . Elle a pour objet des produits provenant de la réaction d'un amine-diol avec une substance polyfonctionnelle , lesdits produits ayant un caractère de résine . Par introduction de groupes cationiques , ladite résine peut être mise sous forme d'une dispersion aqueuse directement utilisable comme additif dans une composition de peinture cationique électrodéposable .

La technique de formation de peinture ou vernis par électrodéposition à partir d'un bain de composition chimique appropriée est connue de l'homme du métier . Il existe beaucoup de références bibliographiques et de brevets sur un tel sujet . A titre illustratif , on peut citer dans le domaine de l'invention les brevets US 4.689.131 et 4.810.535 qui ont été récemment délivrés et qui décrivent des additifs destinés à améliorer l'aspect des revêtements obtenus par électrodéposation . Une telle technique trouve une large application dans l'industrie automobile . En effet , les carrosseries d'automobiles sont très généralement recouvertes d'une peinture ou d'un vernis par électrodéposition .

A l'heure actuelle , on recherche de plus en plus des tôles possédant une bonne résistance à la corrosion, ce qui conduit à sélectionner des alliages de fer particuliers ainsi que des compositions de laques encore améliorées par rapport à celles déjà disponibles . Par exemple , on a constaté que les alliages fer-zinc conviennent bien pour la réalisation de tôles ayant une résistance élevée à la corrosion . On sait néanmoins que ce type de tôles , en particulier, est difficile à traiter par la technique d'électrodéposition , lorsqu'on veut réaliser un revêtement de surface exempt de défauts . On constate notamment l'apparition de piqûres ou de cratères , défauts de surface qui sont dûs à l'application de la différence de potentiel nécessaire à la technique d'électrodéposition ( ou électrophorèse ) . Lorsque les pièces en tôles , comme c'est le cas des carrosseries d'automobiles , présentent des zones difficilement accessibles, on a tendance à augmenter le voltage d'application pour être sûr que l'ensemble de la carrosserie sera muni d'un revêtement convenable . Par là même , on augmente les risques de piqûres , lesquelles sont précisément imputables à de petits arcs électriques , qui partent du substrat métallique et viennent traverser le revêtement en surface .

La présence d'un revêtement d'électrophorèse parfaitement continu est évidemment très souhaitable , non seulement pour la résistance à la corrosion mais aussi pour une propriété habituellement dénommée " résistance au gravillonnage " , qui traduit l'aptitude des zones métalliques de la voiture à bien résister aux projections d'objets les plus divers , auxquelles ces zones peuvent être soumises lors de l'utilisation du véhicule .

On connaît dans l'état de la technique des composés de type amine-diol. De tels composés ont déjà été utilisés , par exemple , pour imprégner les textiles afin de leur redonner du lustre . Cette application a fait l'objet de la demande de brevet publiée EP-11.130. Les produits décrits dans ce document sont des amines-diols peu polymérisés.

Le brevet US 4.001.329 est relatif à un produit de réaction entre une aniline, du formaldéhyde et de la diéthanolamine. Le produit est utilisé pour la production de mousse de polyuréthane. Le composé obtenu est un polyol comportant des groupements hydroxyles libres.

Le brevet US 4.761.502 concerne lui aussi l'obtention d'aminediols dont les groupements hydroxyles ne sont pas substitués. Les produits sont utilisés comme intermédiaires dans la fabrication de colorants.

D'autres applications d'amines substituées par des groupements aliphatiques ou aromatiques sont aussi décrites dans la littérature. On citera ainsi l'utilisation de composés de ce type pour améliorer les performances d'encres pour imprimantes (Chemical Abstracts Vol.97, n°22, 1982, résumé n°184-144 G), dans la fabrication de papier copieur sensible à l'impression (Chemical Abstracts, Vol.79 N°25, 1973, résumé n°151.696 P), ou dans des émulsions cationiques utilisées dans la stabilisation des sols (Chemical Abstracts, Vol.101, n°24, 1984, résumé n° 215, 560 X; Chemical Abstracts, Vol. n°21, 1986, résumé n° 185.507 C).

Le brevet US-4 588.840 quant à lui enseigne la réaction d'un polyalkylène polyol avec une amine aromatique. Ce document indique simplement que le produit de cette réaction peut lui-même réagir avec un isocyanate organique (colonne 1). Il n'y a donc aucune suggestion ou indication d'une possible réaction avec un matériau polyfonctionnel.

Le brevet GB-2.187.449 décrit la réaction d'oxyalkylation de la N-(2-hydroxyalkyl)-aniline. Ce composé est une amine secondaire et non une amine primaire.

On notera en outre que ces deux brevets concernent des applications autres que celles d'additifs pour compositions de peinture.

Il ressort donc de ce qui précède que les produits décrits dans la littérature ne présentent que des poids moléculaires faibles et n'ont pas été utilisés pour préparer, par réaction avec d'autres agents actifs, des additifs pour compositions de peinture.

L'invention a pour objet un additif pouvant être utilisé dans une composition de peinture d'électrodéposition qui, après application sur un substrat métallique, fournit un revêtement ou film ayant une résistance améliorée aux piqûres et à la cratérisation .

Un autre objet de l'invention est de fournir un additif du type ci-dessus , qui améliore l'aspect de film dans un système de peinture cationique électrodéposable , et qui simultanément n'altère pas l'adhérence des couches habituellement utilisées dans le revêtement des tôles , telles que les couches de finition ou les mastics .

Encore un autre objet de l'invention est de fournir un additif pour peinture électrodéposable dont la composition permet de travailler avec des différences de potentiel plus élevées , ce qui procure une meilleure possibilité de revêtir les zones les moins accessibles d'un substrat métallique , tel qu'une carrosserie d'automobile .

Encore un autre objet de l'invention est un additif pour système de peinture cationique électrodéposable qui , dans de nombreux cas , n'utilise pas de solvants , ce qui contribue à la protection de l'environnement , l'électrodéposition pouvant être réalisée sans qu'il soit nécessaire de mettre en oeuvre les précautions qui sont indispensables avec des systèmes comportant un solvant.

Sous sa forme générale, l'invention a pour objet le produit de réaction d'un amine-diol, tel que défini ci-après , et d'une substance polyfonctionnelle capable de réagir avec les groupes hydroxyles dudit amine-diol.

L'amine-diol utilisé pour l'obtention du produit de l'invention résulte lui-même de la réaction d'une amine primaire de formule R-NH₂ dans laquelle R désigne un radical aliphatique ou aromatique, avec l'oxyde de propylène ou un mélange d'un oxydes d'alkylène comprenant en prédominance de l'oxyde de propylène .

En vue de son application dans les systèmes de peintures électrodéposables , il est mis à réagir pour former une résine avec au moins une substance polyfonctionnelle capable d'agir sur ses groupes hydroxyles .

Le groupement R de l'amine comporte généralement 1 à 20 atomes de carbone . Il s'agit de préférence de groupes alkyle ou aralkyle , à chaîne linéaire ou ramifiée . On donne la préférence aux amines dans lesquelles le radical R comporte de 1 à 12 atomes de carbone et est choisi , par exemple , parmi les radicaux méthyle , butyle ou lauryle . Le groupe R peut être également de nature aromatique , par exemple un groupe phényle . Il peut alors comporter de 6 à 18 atomes de carbone , la préférence étant donnée à la gamme de C₆ à C₁₂ environ .

L'autre réactif utilisé pour préparer l'amine-diol de l'invention est un oxyde d'alkylène comprenant en prédominance de l'oxyde de propylène . Cette expression signifie que, pour les besoins de l'invention , c'est-à-dire dans l'application aux systèmes de peinture électrodéposable , l'oxyde d'éthylène , utilisé isolément en réaction avec l'amine R-NH₂ ne serait pas approprié , car il conduirait à un composé ayant des caractéristiques trop élevées de solubilité. C'est pourquoi on utilise le plus avantageusement l'oxyde de propylène . Toutefois , celui-ci peut être mis en oeuvre en mélange avec d'autres oxydes d'alkylène , même avec une faible proportion d'oxyde d'éthylène . L'oxyde de butylène ou l'oxyde d'isobutylène sont théoriquement utilisables , mais on préfère dans ce cas les mélanger avec l'oxyde de propylène . Celui-ci , pour les besoins de l'invention se trouve en prédominance , c'est-à-dire à plus de 50% en poids et de préférence à plus de 80% en poids par rapport au mélange d'oxydes d'alkylène .

Selon un mode de mise en oeuvre préféré de l'invention, on a trouvé que le composé de type amine-diol devait comporter un poids moléculaire compris environ entre 800 et 1500 et encore mieux entre 1000 et 1200. Des essais pratiques ont en effet montré que des additifs provenant d'amine-diol ayant un poids moléculaire supérieur à 1500 environ, par exemple de l'ordre de 2000 , bien qu'offrant une très bonne résistance à la cratérisation, provoquaient une altération de l'adhérence de certaines couches de finition (notamment à base de résines alkydes ). Par ailleurs, des produits provenant d'amine-diol ayant un poids moléculaire inférieur à 800 , environ, par exemple de 500 à 800 ne présentent pas de défauts d'adhérence , mais n'offrent pas une bonne résistance à la cratérisation . Selon l'invention, on a donc trouvé que les fourchettes de poids moléculaires appropriés de l'amine-diol , pour fournir un additif offrant à la fois des qualités de bonne adhérence et de bonne résistance à la cratérisation , se situaient entre 800 et 1500 environ et encore plus avantageusement entre 1000 et 1200.

On conduit la réaction de l'amine primaire R-NH₂ sur l'oxyde d'alkylène pour , dans une première étape , mettre en présence les deux constituants de la réaction dans des conditions douces , c'est-à-dire sans catalyseur et avec une élévation modérée de température ( ne dépassant pas 50°C environ, la température de réaction étant alors par exemple de 40°C ) et avec un léger excès d'amine par rapport à l'oxyde d'alkylène . Cet excès peut aller jusqu'à 2/1 en moles .

On poursuit ensuite la réaction pour allonger la chaîne du composé en mettant en présence des molécules complémentaires d'oxyde d'alkylène avec le produit issu de la première étape , et en présence d'un catalyseur basique , tel que l'hydroxyde de potassium . La réaction est poursuivie jusqu'à l'ajustement du poids moléculaire dans les fourchettes indiquées ci-dessus .

On a d'abord schématiquement illustré la formation de l'amine-diol par réaction de l'amine R-NH₂ sur un oxyde d'alkylène O-Alk. 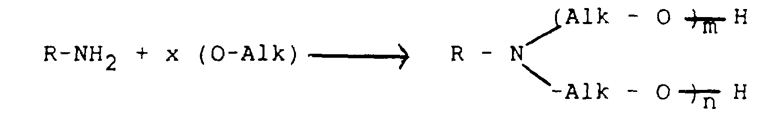 avec m + n = x .

On a également illustré ci-après le schéma réactionnel dans le cas de l'oxyde de propylène . Le mécanisme réactionnel a été simplifié pour montrer la réaction de x moles d'oxyde de propylène jusqu'à l'obtention du composé amine-diol ayant le poids moléculaire voulu. 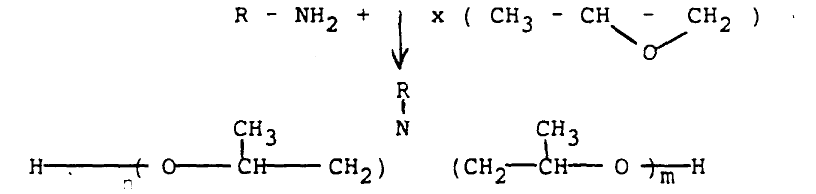 avec m + n = x .

L'amine-diol ainsi obtenu , qui est une monoamine tertiaire , comprend des groupements hydroxyles.L'homme du métier comprendra que le nombre de chaînons oxyde d'alkylène , en particulier oxyde de propylène, variera selon le poids moléculaire à obtenir, et aussi selon l'amine R-NH₂ de base utilisée. En général , le nombre de groupements oxyde de propylène peut varier de 10 à 40.

En vue de son application dans des systèmes de peintures électrodéposables , le composé de type amine-diol est mis à réagir , pour former une résine , avec au moins une substance polyfonctionnelle capable d'agir sur les groupes hydroxyles de l'amine-diol. Il existe de nombreuses substances aptes à remplir cette fonction, les plus accessibles étant les isocyanates , les acides et anhydrides d'acide . Le résultat d'une telle réaction est un allongement de chaîne et une augmentation du poids moléculaire , qui aboutit à une résine laquelle , par neutralisation, est susceptible de constituer une dispersion aqueuse grâce à la formation de sites cationiques .

On utilise avantageusement des diisocyanates tels que le toluène-diisocyanate , l'isophorone-diisocyanate, l'hexaméthylène-diisocyanate , le tétraméthylphényl-isocyanate, le méthylène-diphényl diisocyanate (MDI) et le polyméthylène-polyphényl isocyanate et autres isocyanates analogues déjà connus dans la technique des résines électrodéposables . On peut se reporter à ce sujet au brevet US 4 689 131, déjà cité au début de la présente description. Les conditions réactionnelles impliquent l'utilisation d'un excès d'amine-diol par rapport à l'isocyanate . Si l'on définit les proportions des réactifs par les équivalents de groupes OH par rapport aux groupes isocyanates NCO, le rapport en moles OH/NCO peut être compris entre 2/1 et 4/3 environ, un rapport voisin de 3/2 étant préféré .

Au lieu des isocyanates , on peut utiliser , comme on l'a indiqué ci-dessus, d'autres allongeurs de chaîne, tels que des acides ou anhydrides d'acide , notamment des diacides ou anhydrides de diacide , tels les acides ou anhydrides phtalique et maléique .

Les proportions des amine-diols par rapport aux acides ou anhydrides d'acide seront alors avantageusement de 2 à 4/3 et préférentiellement de 3/2, le rapport étant défini en équivalents de groupes OH par rapport aux groupes CO₂H ( OH/CO₂H en moles).

Cependant , dans la pratique , on donne la préférence aux isocyanates, car d'une manière générale les durées des réactions avec les acides ou anhydrides d'acide sont plus élevées .

Dans certains cas, et notamment si le radical R de l'amine R-NH₂ contient plus d'un ou deux atomes de carbone , il peut être nécessaire , pour assurer une bonne dispersion aqueuse de la résine préparée à partir du composé amine-diol correspondant , d'utiliser conjointement , ainsi qu'il est connu de l'homme du métier , une autre résine, dite de broyage qui facilite la dilution dans l'eau . Cette technique est connue en elle-même et sera illustrée dans les exemples qui suivent .

L'invention procure ainsi un additif empêchant la formation de cratères , présentant une excellente adhérence aux apprêts , aux mastics et aux couches de finition, et conférant aussi une résistance améliorée du film à la formation de piqûres sur des substrats métalliques , en particulier sur des substrats métalliques à base d'alliages hétérogènes , plus spécialement de type fer-zinc , et notamment les substrats prétraités par galvanisation , électrozingage ou plus généralement par dépôt de revêtement zingué . En outre , le produit de l'invention comprend un polymère sans solvant .

L'invention sera encore illustrée , sans être aucunement limitée, par les exemples ci-après .

Sauf indications contraires , toutes les parties sont exprimées en poids .

### EXEMPLE 1:

On a préparé un véhicule pour broyage de pigments , à partir du mélange de constituants suivant:

| Ingrédients | Parties en poids | Solides | Equivalents |
|---|---|---|---|
| Epon 828 * | 531,0 | 531,0 | 2,82 |
| Bisphénol A | 208,0 | 208,0 | 1,82 |
| Iodure de triéthylphényl-phosphonium | 0,53 | | |
| Xylène | 19,1 | | |
| 2-éthyhexanolmono-uréthanne de 2,4-toluènediisocyanate , à 95% dans de la méthylisobutylcétone | 301,4 | 286,3 | |
| Agent de quaternisation ^{⁽¹⁾} | 461,3 | 315,9 | |
| 2-butoxyéthanol | 1001,9 | | |
| | 2523,2 | 1341,2 | |

| | | | |
|---|---|---|---|
| * Résine époxyde obtenue par réaction d'épichlorhydrine et de bisphénol A ayant un équivalent d'époxyde de 188, commercialisée par Shell Chemical Company. | | | |
| (1) On prépare l'agent de quaternisation en faisant réagir 328 parties en poids de 2-éthylhexanolmonouréthanne de 2,4-toluène diisocyanate dans de la méthyléthylcétone à la température ambiante avec 9,2 parties en poids de triméthyléthanolamine . On laisse le mélange jusqu'à réaction exothermique et on le maintient pendant 1 heure à 80°C , puis on ajoute 120,3 parties en poids d'acide lactique , puis 107 parties de butoxyéthanol. Le mélange réactionnel est ensuite agité pendant 1 heure à 65°C , après incorporation de 94 parties en poids d'eau. | | | |

Le catalyseur , le xylène , le Epon 828 et le bisphénol A ont été introduits sous atmosphère d'azote dans un réacteur et chauffés à 150-160°C pour déclencher la réaction exothermique . Au bout d'une heure à 160°C , le mélange réactionnel a été refroidi à 120°C et on a ajouté le 2-éthylhexanolmono-uréthanne de 2,4-toluènediisocyanate , à 95% dans de la méthylisobu- tylcétone . La température du mélange réactionnel a été maintenue à 115°C pendant 1 heure , puis on a ajouté le 2-butoxyéthanol . Le mélange réactionnel a été ensuite refroidi à 85°C, homogénéisé puis additionné de l'agent de quaternisation . La température du mélange réactionnel a été maintenue à 80-85°C jusqu'à obtention d'un indice d'acide d'environ 1. Le mélange réactionnel avait un extrait sec de 53 %.

### EXEMPLE 2:

On a préparé une résine cationique classique à partir du mélange d'ingrédients suivant :

| Ingrédients | Parties en poids | Solides | Equivalents |
|---|---|---|---|
| Epon 828 | 702,2 | 702,2 | 3,735 |
| PCP 200** | 263,4 | 263,4 | 1,000 |
| Bisphénol A | 197,8 | 197,8 | 1,735 |
| Xylène | 61,6 | | |
| Benzyldiméthylamine | 3,8 | | |
| Agent de réticulation isocyanate coiffé ^{²} | 891 | 629,1 | |
| Dicétimine dérivant de diéthylènetriamine et de méthylisobutylcétone (extrait sec 76 % dans la méthylisobutylcétone) | 75,3 | 54,7 | 0,612 |
| N-méthyl-éthanolamine | 59,1 | 59,1 | 0,787 |
| Phénoxypropanol | 126,9 | 11,7 | |
| Tensioactif cationique ^{³} | 29,3 | 11,7 | |
| Acide acétique | 29,5 | | |
| H₂O 36% | 2917,2 | | |
| | 5327,8 | 1918 | |

| | | | |
|---|---|---|---|
| ** PCP 200 : polycaprolactone-diol vendu par Union Carbide Corp. | | | |
| 2) Agent de réticulation polyuréthanne , provenant de la réaction du toluène diisocyanate ( mélange 80/20 isomère 2-4/2-6), avec de l'éthyl-2-hexanol , et traitement du produit avec du triméthylolpropane selon une proportion molaire 3:1. L'agent de réticulation est présent sous forme d'une solution dans du méthoxypropanol à 70% d'extrait sec . | | | |
| 3) On prépare le tensioactif cationique en mélangeant 120 parties d'une alkylimidazoline commercialisée par Geigy Industrial Chemical sous la marque GEIGY Amine C, 120 parties en poids d'un alcool acétylénique commercialisé par Ast Products and Chemicals Inc. sous la marque Surfynol 104, 120 parties en poids de 2-butoxyéthanol , 221 parties en poids d'eau désionisée et 19 parties d'acide acétique glacial. | | | |

Le Epon 828, le PCP 200 et le xylène ont été introduits dans un réacteur et chauffés sous barbotage d'azote à 210°C. Le mélange réactionnel a été maintenu au reflux pendant 1/2 heure pour éliminer l'eau. Le mélange réactionnel a été refroidi à 150°C , et on a ajouté le bisphénol A et 1,6 parties du catalyseur benzyldiméthylamine . Le mélange réactionnel a été chauffé à 150-190°C et maintenu à cette température pendant environ 1,5 heures , puis refroidi à 130°C. La partie restante du catalyseur benzyldiméthylamine a été ajoutée , et le mélange réactionnel a été maintenu à 130°C pendant environ 3 heures jusqu'à ce qu'on obtienne une viscosité réduite ( solution à extrait sec résine 50% dans du méthoxypropanol), correspondant à l'indice P dans l'échelle Gardner-Holdt .

L'agent de réticulation au polyuréthanne, la dicétimine et la méthyléthanolamine ont été ensuite introduits, et la température du mélange réactionnel a été portée à 110°C et maintenue à cette valeur pendant 1 heure.

Le phénoxypropanol a été ajouté, et le mélange réactionnel a été dispersé dans de l'eau après avoir été ajouté à un mélange d'acide acétique, d'eau désionisée et du tensioactif cationique³. Cette dispersion a été diluée à 36% d'extrait sec avec de l'eau désionisée .

### EXEMPLE 3:

On a préparé à partir du mélange d'ingrédients suivant une résine cationique classique débarrassée des solvants par distillation.

| Ingrédients | Parties en poids | Solides | Equivalents |
|---|---|---|---|
| Epon 828 | 682,3 | 682,3 | 3,629 |
| Bisphénol A | 197,7 | 197,7 | 1,732 |
| Polyétherdiol ^{¹} | 238,7 | 238,7 | 1 |
| Méthylisobutyl cétone | 58,8 | | |
| Benzyldiméthylamine (total) | 2,3 | | |
| Agent de réticulation polyuréthanne | 975,4 | 682,8 | |
| Dicétimine dérivant de diéthylènetriamine et de méthylisobutylcétone (extrait sec 73% dans la méthylisobutylcétone) | 76,1 | 55,5 | |
| N-méthyléthanolamine | 64,8 | 64,8 | |
| Phénoxypropanol | 96,2 | | |
| Acide lactique | 70 | 61,6 | |
| H₂O | 3548 | | |
| | 6010,3 | 1983,4 | |

| | | | |
|---|---|---|---|
| 1) Diol obtenu par réaction du bisphénol A avec de l'oxyde d'éthylène selon une stoechiométrie telle que le produit final ait un poids équivalent d'hydroxyle d'environ 239. | | | |

2) Agent de réticulation polyuréthanne provenant de toluène diisocyanate (mélange 80:20 des isomères 2-4/2-6) avec de l'hexoxyéthanol (éther monohexylique de l'éthylèneglycol) , dilué à 70% dans la méthylisobutylcétone .

L'Epon 828, le polyétherdiol, le bisphénol A et la méthylisobutylcétone ont été introduits dans un réacteur et chauffés avec barbotage d'azote à 200°C . Le mélange réactionnel a été maintenu au reflux pendant environ 1/2 heure pour éliminer l'eau. Le mélange réactionnel a été refroidi à 150°C , et on a ajouté 1,1 partie du catalyseur benzyldiméthylamine . Le mélange réactionnel a été chauffé à 150-190°C et maintenu à cette température pendant environ 1 heure , puis refroidi à 130°C avant d'ajouter 1,2 parties de benzyldiméthylamine et maintien pendant environ 3 heures jusqu'à ce qu'on arrive à une viscosité Gardner-Holdt réduite (résine extrait sec 50% dans le méthoxypropanol) correspondant à l'indice R.

L'agent de réticulation polyuréthanne, la dicétimine et la N-méthyl-éthanolamine ont été ensuite introduits dans le mélange réactionnel, et la température a été portée à 110°C et maintenue à cette valeur pendant 1 heure.

Le phénoxypropanol a été ajouté, et le mélange réactionnel a été dispersé dans l'eau par addition dans un mélange d'acide lactique et d'eau désionisée. Cette dispersion a été diluée à un extrait sec de 33% avec de l'eau désionisée, et elle a été distillée sous vide pour éliminer les solvants organiques volatils de façon à donner une dispersion ayant une teneur en extrait sec de 38%.

### EXEMPLE 4:

On a préparé à partir du mélange d'ingrédients suivants un méthylamino-polyoxypropylènediol :

| Ingrédients | Parties en poids | Solides | Equivalents |
|---|---|---|---|
| Méthylamine à 40% dans l'eau | 77,5 | 31 | 1 |
| Oxyde de propylène | 115,7 | 115,7 | 1,995 |
| KOH | 1,1 | | |
| Oxyde de propylène | 866,2 | 866,2 | 14,935 |
| Acide acétique | 1,1 | | |
| | | 1012,9 | |

Dans un réacteur sec et propre muni d'un agitateur , d'un chauffage et d'un refroidissement , et capable de conserver une pression interne , sous enceinte étanche , de 1034 kPa (150 psi) , on introduit la solution de méthylamine et on ferme le réacteur d'une manière étanche. La température est élevée à 40°C et, à cette température , on ajoute lentement l'oxyde de propylène . La réaction exothermique est régulée par refroidissement , et par le taux d'addition de l'oxyde de propylène . La température de réaction est ensuite maintenue à 40°C . Quand l'oxyde a été entièrement introduit dans le réacteur , le mélange réactionnel est maintenu pendant 1 heure à 40°C , après quoi on réalise un prélèvement pour le dosage de l'amine tertiaire et la détermination de l'équivalent de neutralisation (94% minimum, et respectivement 180-200).

Quand les analyses concernant l'amine tertiaire et l'équivalent de neutralisation sont conformes , le mélange réactionnel est chauffé à 100°C , et l'eau est chassée par distillation sous vide sous une pression inférieure à 1333 Pa (10 mm Hg). L'élimination par distillation est interrompue quand la teneur en eau , mesurée par la méthode de Karl Fischer, est inférieure à 0,15 %.

Quand la teneur en eau est conforme , le mélange réactionnel est refroidi à 40°C et on ajoute le catalyseur KOH. Puis le réacteur est purgé à l'azote (trois fois ). Le mélange réactionnel est ensuite chauffé à 120°C, et on ajoute lentement de l'oxyde de propylène . On maintient la température à 120-125°C en agissant sur le taux d'addition de l'oxyde de propylène et en refroidissant le réacteur . Après introduction complète de l'oxyde , le mélange réactionnel est maintenu pendant 1 heure à 120-125°C , puis on effectue des prélèvements pour déterminer l'équivalent de neutralisation et doser l'amine tertiaire . Si nécessaire , on peut ajouter de l'oxyde de propylène et le faire réagir , pour ajuster la réaction .

Une fois que le produit est jugé conforme , il est refroidi à 40°C et neutralisé à l'acide acétique .

| | |
|---|---|
| Equivalent de neutralisation | 1000-1250 |
| Amine tertiaire | 94 % minimum |
| Poids équivalent d'hydroxyle | 430-510 |

### EXEMPLE 5:

On a préparé à partir du mélange d'ingrédients suivant un laurylaminopolyoxypropylènediol.

| Ingrédients | Parties en poids | Solides | Equivalents |
|---|---|---|---|
| Laurylamine | 171 | 171 | 1 |
| Oxyde de propylène | 115,7 | 115,7 | 1,995 |
| KOH | 1,1 | | |
| Oxyde de propylène | 866,2 | 866,2 | 14,935 |
| Acide acétique | 1,1 | | |
| Total | 1152,9 | | |

Le mode opératoire est analogue à celui de l'exemple 4 , en ajustant l'équivalent de neutralisation et le poids d'hydroxyle .

### EXEMPLE 6:

On a préparé à partir du mélange d'ingrédients suivant un butylaminopolyoxypropylènediol:

| Ingrédients | Parties en poids | Solides | Equivalents |
|---|---|---|---|
| Butylamine | 73 | 73 | 1 |
| Oxyde de propylène | 115,7 | 115,7 | 1,995 |
| KOH | 1,1 | | |
| Oxyde de propylène | 866,2 | 866,2 | 14,935 |
| Acide acétique | 1,1 | | |
| | | 1054,9 | |

Le mode opératoire est analogue à celui de l'exemple 4, en ajustant l'équivalent de neutralisation et le poids équivalent d'hydroxyle.

### EXEMPLE 7:

On a préparé à partir du mélange d'ingrédients suivant un phénylaminopolyoxypropylènediol :

| Ingrédients | Parties en poids | Solides | Equivalents |
|---|---|---|---|
| Phénylamine | 91 | 91 | 1 |
| Oxyde de propylène | 115,7 | 115,7 | 1,995 |
| KOH | 1,1 | | |
| Oxyde de propylène | 866,2 | 866,2 | 14,935 |
| Acide acétique | | 1,1 | |
| | | 1072,9 | |

Le mode opératoire est analogue à celui de l'exemple 4 , en ajustant l'équivalent de neutralisation et le poids équivalent d'hydroxyle .

### EXEMPLE 8 :

On a préparé à partir du mélange d'ingrédients suivant un polyuréthanne-polyaminodiol:

| Ingrédients | Parties en poids | Solides | Equivalents |
|---|---|---|---|
| Méthylaminopolyoxy propylènediol de l'exemple 4 | 2712 | 2712 | 6 (OH) |
| MIBK, méthylisobutylcétone | 135,6 | | |
| Dilaurate de dibutylétain | 0,5 | | |
| Diisocyanate de toluène (80/20, isomères 2,4/2,6 | 348 | 348 | 4 (NCO) |
| Acide acétique | 180 | | 3,0 |
| Eau désionisée | 5123,9 | | |
| | 8500 | 3600 | |

Le méthylaminopolyoxypropylènediol et la méthylisobutylcétone ont été introduits sous couverture d'azote dans un réacteur et chauffés à 120°C . A 120°C, le mélange réactionnel est mis sous vide pour éliminer la méthylisobutylcétone et pour sécher l'aminediol. Quand la distillation est terminée, on interrompt l'arrivée de vide à l'aide d'une couverture d'azote et on abaisse la température à 60°C.

A 60°C , le catalyseur est ajouté, puis on commence l'addition du diisocyanate de toluène . La réaction exothermique est thermostatée ( par le taux d'addition du diisocyanate de toluène et refroidissement ) à une température maximale de 80°C . Quand l'addition est terminée, le produit est maintenu pendant 1 heure à 80°C jusqu'à ce qu'aucun groupe NCO ne soit décelable aux infrarouges .

Le produit est ensuite dissous dans un mélange d'acide acétique et d'eau désionisée.

### EXEMPLE 9:

On a préparé à partir du mélange d'ingrédients ci-après un polyuréthannepolyaminodiol:

| Ingrédients | Parties en poids | Solides |
|---|---|---|
| Méthylaminopolyoxypropylène diol de l'exemple 4 | 2712 | 2712 |
| MIBK, méthylisobutyl cétone | 135,6 | |
| Dilaurate de dibutyl étain | 0,5 | |
| Diisocyanate de toluène (80/20, isomères 2,4/2,6) | 348 | 348 |
| Véhicule de broyage de l'exemple 1 | 6018 | 3190 |
| Acide acétique | 130 | |
| Eau désionisée | 8153 | |
| | | 6250 |

Le mode opératoire est le même qu'à l'exemple 8, à ceci près que le mélange comprend en outre le véhicule de broyage de l'exemple 1.

A cet effet le produit est mélangé à l'acide acétique et audit véhicule de broyage et finalement dispersé dans de l'eau désionisée.

### EXEMPLE 10

On a préparé à partir du mélange d'ingrédients suivant un polyuréthannepolyaminodiol.

| Ingrédients | Parties en poids | Solides | Equivalents |
|---|---|---|---|
| Laurylaminopolyoxypropylènediol de l'exemple 5 | 3060 | 3060 | 6 (OH) |
| MIBK, méthylisobutylcétone | 153 | | |
| Dilaurate de dibutylétain | 0,5 | | |
| Diisocyanate de toluène (80/20 , isomères 2-4/2-6) | 348 | 348 | 4 (NCO) |
| Acide acétique | 165 | | 2,75 |
| Véhicule de broyage de l'exemple 1 | 6430 | 3408 | |
| H₂O pour extrait sec 36% | 8930 | | |
| | 18933 | | 6816 |

Le mode opératoire est analogue à celui décrit dans l'exemple 9 .

### EXEMPLE 11:

On a préparé à partir du mélange d'ingrédients suivant un polyuréthannepolyaminodiol:

| Ingrédients | Parties en poids | Solides | Equivalents |
|---|---|---|---|
| Butylaminopolyoxypropylènediol de l'exemple 6 | 3114 | 3114 | 6 (OH) |
| Méthylisobutylcétone | 155,7 | | |
| Dilaurate de dibutyl étain | 0,5 | | |
| Diisocyanate de toluène (80/20 , isomères 2-4/2-6) | 348 | 348 | 4 (NCO) |
| Acide acétique | 148 | | |
| Véhicule de broyage de l'exemple 1 | 6811 | 3610 | |
| Eau désionisée | 9223 | | |
| | 19644 | 7072 | |

Le mode opératoire est analogue à celui décrit dans l'exemple 9.

### EXEMPLE 12:

On a préparé à partir du mélange d'ingrédients suivant un polyuréthannepolyaminodiol.

| Ingrédients | Parties en poids | Solides | Equivalents |
|---|---|---|---|
| Phénylaminopolyoxy propylènediol de l'exemple 7 | 3468 | 3468 | 6 (OH) |
| Méthylisobutylcétone | 173,4 | | |
| Dilaurate de dibutylétain | 0,5 | | |
| Diisocyanate de toluène (80/20 , isomères 2,4/2,6) 3,45(NCO) | 300,6 | | 300,6 |
| Acide acétique | 123 | | 2,06 |
| Véhicule de broyage de l'exemple 1 | 7343 | 3821,6 | |
| Eau désionisée | 10385,4 | | |
| Total | 21620 | 7783,2 | |

Le mode opératoire est analogue à celui décrit dans l'exemple 9.

### EXEMPLE 13 (comparaison)

On a préparé à partir du mélange d'ingrédients suivant un produit d'addition polyoxyalkylènepolyaminepolyépoxyde tel que décrit dans l'exemple 1 du brevet US N° 4 432 850.

| Ingrédients | Parties en poids |
|---|---|
| Jeffamine D2000 ^{⁽¹⁾} | 1415,9 |
| Epon 1001^{⁽²⁾} | 489,1 |
| 2-butoxyéthanol | 179,8 |
| Acide acétique | 29,5 |
| H₂O pour extrait sec 36% | 3178 |
| Total | 5292 |

| | |
|---|---|
| 1) La Jeffamine D2000 est une polyoxypropylènediamine ayant une masse moléculaire de 2000, commercialisée par Jefferson Chemical Company ou Texaco. | |
| 2) L'Epon 1001 est un éther polyglycidylique du bisphénol A ayant un équivalent d'époxyde de 523 , disponible auprès de Shell Chemical Company. | |

La Jeffamine D2000 a été introduite dans un réacteur sous atmosphère d'azote et chauffée à 90°C , puis on a ajouté une solution d'Epon 1001 dans le butoxyéthanol . Le mélange réactionnel a été chauffé à 110°C et maintenu pendant 2 heures. Le mélange réactionnel a été dispersé dans de l'acide acétique et de l'eau désionisée.

### EXEMPLE 14:

On a préparé une pâte pigmentaire à partir du mélange d'ingrédients suivant .

| | Parties en poids | Equivalents |
|---|---|---|
| Dioxyde de titane | 44,42 | 44,42 |
| Silicate de plomb | 2,9 | 2,9 |
| Noir de carbone | 0,37 | 0,37 |
| Véhicule de broyage de l'exemple 1 | 18,50 | 9,8 |
| Eau désionisée | 27,51 | |
| Pâte de catalyseur ^{⁽¹⁾} | 6,3 | 2,52 |
| Total | 100 | 60,0 |

| | | |
|---|---|---|
| (1) Le catalyseur oxyde de dibutylétain a été dispersé dans le véhicule de broyage de l'exemple 1 comme suit : 28,3 parties du véhicule de broyage de l'exemple 1 , 25 parties en poids d'oxyde de dibutylétain et 46,7 parties en poids d'eau désionisée. | | |

Les ingrédients ci-dessus ont été broyés dans un broyeur jusqu'à la finesse Hegman n°7.

### Compositions de revêtement pouvant être déposées par électrodéposition cationique.

Les exemples 15 à 24 ci-après concernent des compositions de revêtement pouvant être déposées par électrodéposition cationique , contenant les nouveaux additifs selon l'invention, destinés à améliorer l'aspect superficiel sans affecter d'une manière indésirable l'adhérence, ainsi que des additifs de comparaison.

Les compositions ont été déposées par électrodéposition cathodique sur des panneaux en acier ayant subi un traitement préalable au phosphate de zinc. Les revêtements électrodéposés ont durci à température élevée, et le revêtement durci a fait l'objet d'une évaluation de son aspect superficiel . Les revêtements électrodéposés et durcis ont été ensuite revêtus de différentes compositions de revêtement alkyde et polyester, et la couche de couverture a fait l'objet d'une évaluation de son adhérence à la couche de fond obtenue par électrodéposition . L'épaisseur du film sec de la couche de couverture était d'environ 35-40µm . A titre de comparaison, on a aussi évalué des compositions sans additif . Les résultats des essais sont récapitulés sur le tableau I ci-après .

### EXEMPLE 15

A titre de témoin, on a préparé en mélangeant les ingrédients suivants un bain d'électrodéposition cationique sans additif :

| Ingrédients | Parties en poids |
|---|---|
| Résine pour électrodéposition cationique de l'exemple 2 | 988 |
| Pâte pigmentaire de l'exemple 14 | 157 |
| Eau désionisée | 1855 |
| TOTAL | 3000 |

Des panneaux en acier ayant subi un traitement préalable au phosphate de zinc ont été revêtus par électrophorèse cathodique dans le bain d'électrodéposition à 200 V à une température de 24°C . Le temps de déposition a été calculé de façon à donner après durcissement une épaisseur de film de 20-23µm ; dans cet exemple , ce temps a été de 3 min 40 sec .

Ces panneaux ont été utilisés pour contrôler l'adhérence de la couche de couverture et la résistance à la formation des cratères ; dans ce cas , avant cuisson des panneaux , on a placé des gouttes d'huile , avec une seringue, sur le film électrodéposé non durci.

Pour évaluer la résistance du film à la formation des cratères en l'absence d'un polluant sévère, comme l'huile, on a revêtu , dans le bain de résine , un acier laminé à froid type ACT, commercialisé par Advanced Coating Technologies Inc., et possédant une surface très lisse.

### EXEMPLE 16

A titre de témoin , on a préparé en mélangeant les ingrédients suivants un bain d'électrodéposition cationique sans additif .

| Ingrédients | Parties en poids |
|---|---|
| Résine d'électrodéposition cationique de l'exemple 2 | 889 |
| Résine véhicule de broyage de pigment de l'exemple 1 | 68 |
| Pâte pigmentaire de l'exemple 14 | 157 |
| Eau désionisée | 1188 |
| TOTAL | 3000 |

Les panneaux ont reçu un revêtement par électrophorèse comme décrit dans l'exemple 15. Le temps de déposition sur des panneaux en acier ayant subi un traitement préalable au phosphate de zinc a été de 2 min 40 sec .

### EXEMPLE 17:

On a préparé , en mélangeant les ingrédients suivants , un bain d'électrodéposition cationique contenant le produit d'addition de l'exemple 9.

| Ingrédients | Parties en poids |
|---|---|
| Résine d'électrodéposition cationique de l'exemple 2 | 790 |
| Pâte pigmentaire de l'exemple 14 | 157 |
| Eau désionisée | 1855 |
| Additif de l'exemple 9 | 198 |
| TOTAL | 3000 |

Les panneaux ont reçu un revêtement par électrophorèse comme décrit dans l'exemple 15. Le temps de déposition sur des panneaux en acier ayant subi un traitement préalable au phosphate de zinc a été de 2 min 30 sec .

### EXEMPLE 18:

On a préparé en mélangeant les ingrédients suivants un bain d'électrodéposition cationique contenant le produit d'addition de l'exemple 10.

| Ingrédients | Parties en poids |
|---|---|
| Résine d'électrodéposition cationique de l'exemple 2 | 790 |
| Pâte pigmentaire de l'exemple 14 | 157 |
| Eau désionisée | 1855 |
| Additif de l'exemple 10 | 198 |
| TOTAL | 3000 |

Les panneaux ont reçu un revêtement par électrophorèse comme décrit dans l'exemple 15. Le temps de déposition sur des panneaux en acier ayant subi un traitement préalable au phosphate de zinc a été de 2 min 30 sec .

### EXEMPLE 19:

On a préparé en mélangeant les ingrédients suivants un bain d'électrodéposition cationique contenant le produit d'addition de l'exemple 11.

| Ingrédients | Parties en poids |
|---|---|
| Résine d'électrodéposition cationique de l'exemple 2 | 790 |
| Pâte pigmentaire de l'exemple 14 | 157 |
| Eau désionisée | 1855 |
| Additif de l'exemple 10 | 198 |
| TOTAL | 3000 |

Les panneaux ont reçu un revêtement par électrophorèse comme décrit dans l'exemple 15. Le temps de déposition a été de 2 min 30 sec .

### EXEMPLE 20:

On a préparé en mélangeant les ingrédients suivants un bain d'électrodéposition cationique contenant le produit d'addition de l'exemple 12.

| Ingrédients | Parties en poids |
|---|---|
| Résine d'électrodéposition cationique de l'exemple 2 | 790 |
| Pâte pigmentaire de l'exemple 14 | 157 |
| Eau désionisée | 1855 |
| Additif de l'exemple 12 | 198 |
| TOTAL | 3000 |

### EXEMPLE 21

On a préparé en mélangeant les ingrédients suivants un bain d'électrodéposition cationique contenant le produit d'addition de l'exemple 13.

| Ingrédients | Parties en poids |
|---|---|
| Résine d'électrodéposition cationique de l'exemple 2 | 790 |
| Pâte pigmentaire de l'exemple 14 | 157 |
| Eau désionisée | 1855 |
| Additif de l'exemple 13 | 198 |
| TOTAL | 3000 |

### EXEMPLE 22 :

A titre de témoin, on a préparé à partir du mélange d'ingrédients suivants un bain d'électrodépoposition cationique sans additif .

| Ingrédients | Parties en poids |
|---|---|
| Résine pour électrodéposition cationique de l'exemple 3 | 1224 |
| Pâte pigmentaire de l'exemple 14 | 375 |
| Eau désionisée | 1401 |
| TOTAL | 3000 |

Des panneaux prérevêtus d'alliage fer-zinc ("Galva- neal" ) , commercialisés par Advanced Coating Technologies Inc. sous la marque CHR HD6 60/Ad , ont été revêtus par électrophorèse sous différentes tensions pour évaluer la résistance du film à la microrupture (que l'on décrit aussi sous l'expression " piqûres" ) . Pour tous les panneaux revêtus sous une tension de 180 à 300 V , la température du bain était de 28°C , et le temps de déposition a été ajusté pour obtenir une épaisseur de 21 µm . Les résultats sont présentés sur le tableau II.

### EXEMPLE 23 :

On a préparé en mélangeant les ingrédients ci-dessous un bain d'électrodéposition cationique contenant le produit d'addition de l'exemple 8.

| Ingrédients | Parties en poids |
|---|---|
| Résine d'électrodéposition cationique de l'exemple 3 | 1101 |
| Pâte pigmentaire de l'exemple 14 | 375 |
| Eau désionisée | 1395 |
| Additif de l'exemple 8 | 129 |
| TOTAL | 3000 |

Les mêmes panneaux que ceux définis à l'exemple 22 ont été revêtus par électrophorèse selon le mode opératoire décrit à l'exemple 15.

### EXEMPLE 24 :

A titre de témoin, on a préparé en mélangeant les ingrédients suivants un bain d'électrodéposition cationique contenant le produit d'addition de l'exemple 13.

| Ingrédients | Parties en poids |
|---|---|
| Résine d'électrodéposition cationique de l'exemple 3 | 1101 |
| Pâte pigmentaire de l'exemple 14 | 375 |
| Eau désionisée | 1395 |
| Additif de l'exemple 13 | 129 |
| TOTAL | 3000 |

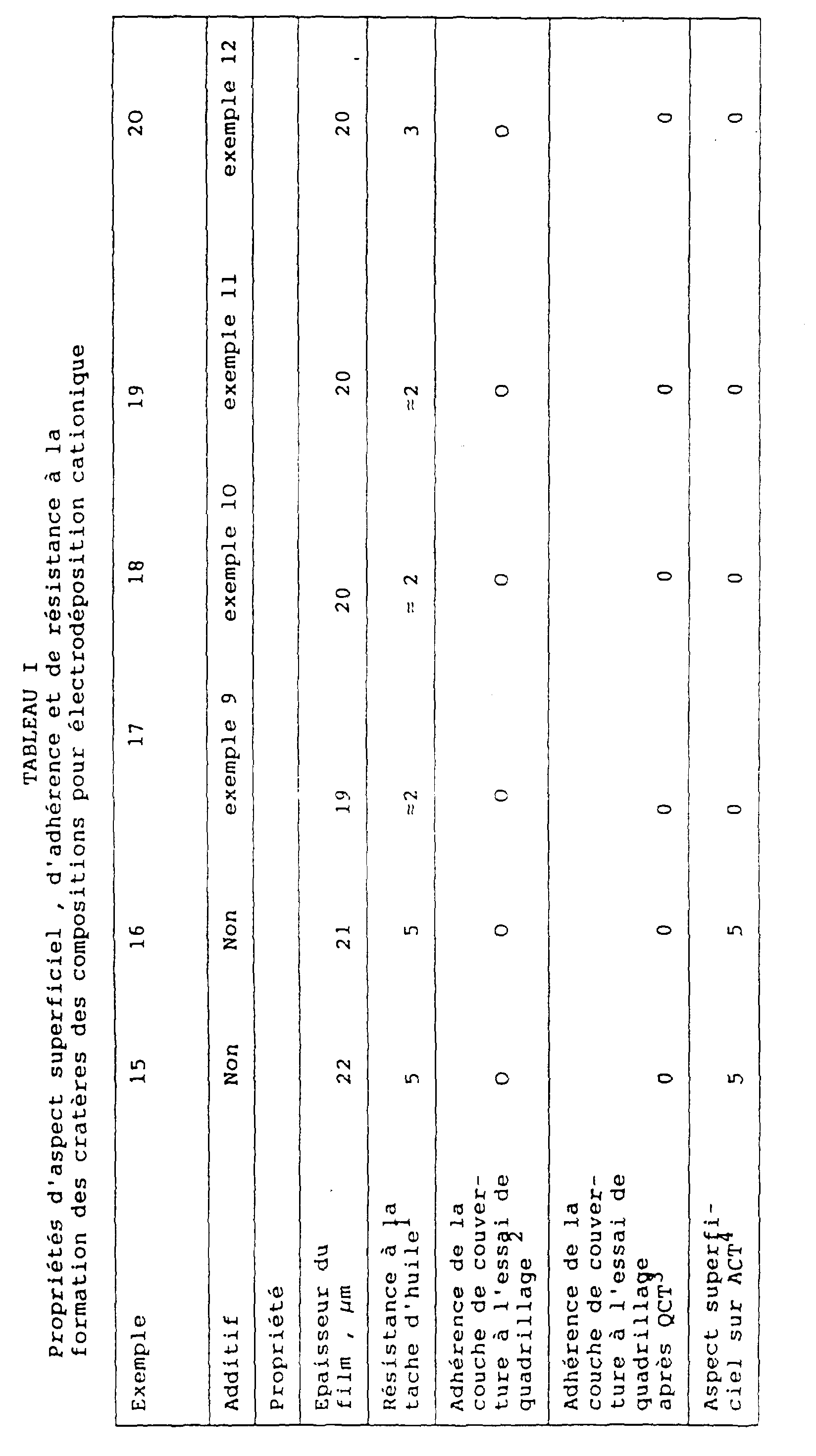

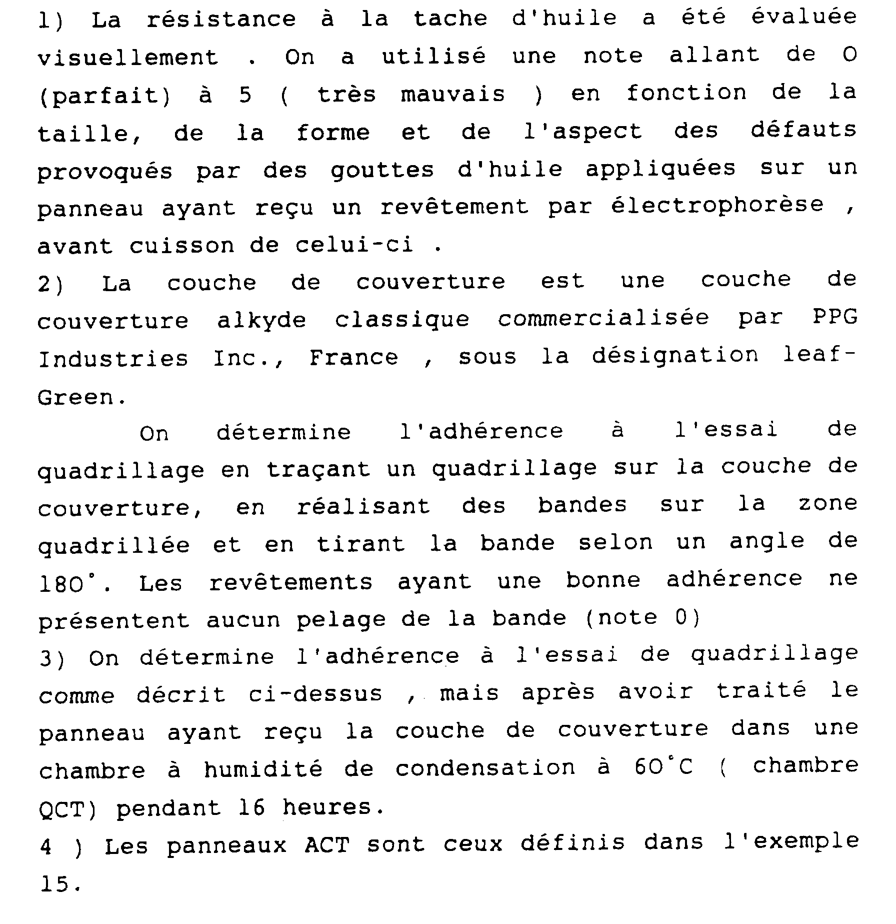

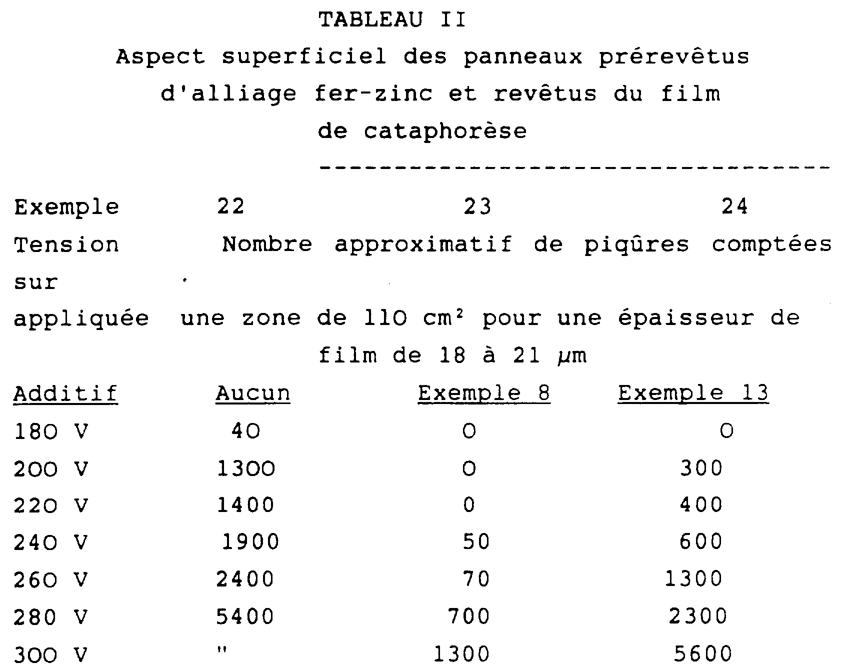

Ainsi qu'on le voit sur le Tableau I d'après les résultats des essais effectués avec les compositions de l'invention ( exemples 17 à 20 , avec les additifs respectifs des exemples 9 à 12 ) :
- la résistance à la tache d'huile est nettement améliorée ,
- l'aspect de la couche de couverture est parfait, avec absence de cratères ( note O sur ACT).
Ces résultats sont obtenus sans perte des propriétés d'adhérence intercouches comme le montrent les essais d'adhérence (notes O). Ceci n'est pas le cas si l'on utilise comme additif le produit de l'exemple 13 de la technique antérieure.

Egalement , on voit d'après le Tableau II que l'utilisation d'un additif selon l'invention ( exemple 8 ) permet de travailler avec des tensions d'application de 220 volts sans apparition de piqûres, ce qui n'est pas le cas du témoin sans additif ni de la composition contenant un additif de l'art antérieur (exemple 13 ) .

## Revendications

1. Composition de peinture cationique électrodéposable contenant le produit de réaction de:
(a) un aminediol provenant de la réaction d'une amine primaire de formule R-NH₂, dans laquelle R désigne un radical aliphatique ou aromatique, avec l'oxyde de propylène ou un mélange d'oxydes d'alkylène comprenant en prédominance de l'oxyde de propylène et de
(b) au moins une substance polyfonctionnelle, capable d'agir sur les groupes hydroxyles dudit aminediol.

2. Composition selon la revendication 1 caractérisée en ce que le groupe R de l'amine R-NH₂ comporte de 1 à 20 atomes de carbone.

3. Composition selon l'une des revendications 1 et 2, caractérisée en ce que le groupe R est un groupement alkyle, ou aralkyle à chaîne linéaire ou ramifiée.

4. Composition selon l'une des revendications 1 à 3, caractérisée en ce que le groupe R comporte de 1 à 12 atomes de carbone.

5. Composition selon l'une des revendications 1 à 4, caractérisée en ce que le groupe R est choisi parmi les radicaux méthyle, butyle ou lauryle.

6. Composition selon l'une des revendications 1 à 4, caractérisée en ce que le groupe R est de nature aromatique, auquel cas il peut alors comporter de 6 à 18 atomes de carbone, et préférentiellement de 6 à 12 atomes de carbone.

7. Composition selon la revendication 6, caractérisée en ce que R est un groupe phényle.

8. Composition selon l'une des revendications 1 à 7, caractérisée en ce que l'oxyde de propylène se trouve dans des proportions supérieures à 50 % en poids , et de préférence supérieures à 80 % en poids par rapport au mélange d'oxydes d'alkylène .

9. Composition selon l'une des revendications 1 à 8 , caractérisée en ce que l'amine-diol (a) possède un poids moléculaire compris entre environ 800 et 1500 et de préférence compris entre 1000 et 1200.

10. Composition selon l'une des revendications 1 à 9 , caractérisée en ce que ladite substance polyfonctionnelle (b) est choisie parmi les isocyanates , les acides et les anhydrides d'acides .

11. Composition selon la revendication 10 , caractérisée en ce que les isocyanates sont choisis parmi les diisocyanates , tels que le toluène diisocyanate , l'isophorone diisocyanate, l'hexaméthylène diisocyanate et le tétraméthylphényl-isocyanate.

12. Composition selon la revendication 10 , caractérisée en ce que les diacides ou anhydrides de diacides sont les acides ou anhydrides phtaliques et maléiques .

13. Composition selon l'une quelconque des revendications 1 à 11 , caractérisée en ce que pour la réaction de l'amine-diol (a) sur la substance polyfonctionnelle (b) , le rapport OH/NCO exprimé en équivalents molaires , des groupes hydroxyles de l'amine-diol (a) aux groupes isocyanates de la substance (b) est compris entre 2/1 et 4/3 environ .

14. Composition selon la revendication 13 caractérisée en ce que le rapport OH/NCO est voisin de 3/2.

15. Composition selon l'une des revendications 1 à 10 et 12 , caractérisée en ce que pour la réaction de l'amine-diol (a) sur la substance polyfonctionnelle (b) , le rapport OH/CO₂H exprimé en équivalents molaires des groupes hydroxyles de l'amine-diol (a) aux groupes acides ou anhydriques de la substance (b) est compris entre 2/1 et 4/3 environ et est préférentiellement voisin de 3/2.

16. Composition selon l'une quelconque des revendications 1 à 15 , caractérisée en ce qu'elle contient en outre une résine de broyage facilitant la dilution dans l'eau.

## Patentansprüche

1. Kationisch elektrolytisch abscheidbare Lackzusammensetzung, umfassend das Reaktionsprodukt aus:
(a) einem Amindiol, das aus der Umsetzung eines primären Amins der Formel R-NH₂, in der R einen aliphatischen oder aromatischen Rest bezeichnet, mit Propylenoxid oder einer Mischung von überwiegend Propylenoxid umfassenden Alkylenoxiden stammt, und
(b) wenigstens einer polyfunktionellen Substanz, die auf die Hydroxylgruppen des Amindiols einwirken kann.

2. Zusammensetzung nach Anspruch 1, dadurch gekennzeichnet, daß die Gruppe R des Amins R-NH₂ 1 bis 20 Kohlenstoffatome umfaßt.

3. Zusammensetzung nach einem der Ansprüche 1 und 2, dadurch gekennzeichnet, daß die Gruppe R eine Alkyl- oder Aralkylgruppe mit einer unverzweigten oder verzweigten Kette ist.

4. Zusammensetzung nach einem der Ansprüche 1 bis 3, dadurch gekennzeichnet, daß die Gruppe R 1 bis 12 Kohlenstoffatome umfaßt.

5. Zusammensetzung nach einem der Ansprüche 1 bis 4, dadurch gekennzeichnet, daß die Gruppe R aus dem Methyl-, Butyl- oder Laurylrest ausgewählt ist.

6. Zusammensetzung nach einem der Ansprüche 1 bis 4, dadurch gekennzeichnet, daß der Rest R aromatischer Natur ist, wobei er 6 bis 18 Kohlenstoffatome und vorzugsweise 6 bis 12 Kohlenstoffatome umfassen kann.

7. Zusammensetzung nach Anspruch 6, dadurch gekennzeichnet, daß der Rest R eine Phenylgruppe ist.

8. Zusammensetzung nach einem der Ansprüche 1 bis 7, dadurch gekennzeichnet, daß das Propylenoxid in den Zusammensetzungen mit einem Anteil von mehr als 50 Gew.-%, vorzugsweise von mehr als 80 Gew.-% vorhanden ist, bezogen auf die Alkylenoxid-Mischung.

9. Zusammensetzung nach einem der Ansprüche 1 bis 8, dadurch gekennzeichnet, daß das Amindiol (a) eine Molmasse zwischen etwa 800 und 1500 und vorzugsweise zwischen 1000 und 1200 aufweist.

10. Zusammensetzung nach einem der Ansprüche 1 bis 9, dadurch gekennzeichnet, daß die polyfunktionelle Substanz (b) aus Isocyanaten, Säuren und Säureanhydriden ausgewählt ist.

11. Zusammensetzung nach Anspruch 10, dadurch gekennzeichnet, daß die Isocyanate aus Diisocyanaten wie Toluoldiisocyanat, Isophorondiisocyanat, Hexamethylendiisocyanat und Tetramethylphenylisocyanat ausgewählt sind.

12. Zusammensetzung nach Anspruch 10, dadurch gekennzeichnet, daß die zweibasigen Säuren oder die Anhydride der zweibasigen Säuren Phthalsäure und Maleinsäure oder deren Anhydride sind.

13. Zusammensetzung nach einem der Ansprüche 1 bis 11, dadurch gekennzeichnet, daß für die Umsetzung des Amindiols (a) mit der polyfunktionellen Substanz (b) das Verhältnis OH/NCO, ausgedrückt in Moläquivalenten, zwischen den Hydroxylgruppen des Amindiols (a) und den Isocyanatgruppen der Substanz (b) etwa 2/1 bis 4/3 beträgt.

14. Zusammensetzung nach Anspruch 13, dadurch gekennzeichnet, daß das Verhältnis OH/NCO ungefähr 3/2 beträgt.

15. Zusammensetzung nach einem der Ansprüche 1 bis 10 und 12, dadurch gekennzeichnet, daß für die Umsetzung des Amindiols (a) mit der polyfunktionellen Substanz (b) das Verhältnis OH/CO₂H, ausgedrückt in Moläquivalenten, zwischen den Hydroxylgruppen des Amindiols (a) und den Säure- oder der Anhydridgruppen der Substanz (b) etwa 2/1 bis 4/3 und vorzugsweise etwa 3/2 beträgt.

16. Zusammensetzung nach einem der Ansprüche 1 bis 15, dadurch gekennzeichnet, daß sie darüber hinaus ein Dispergierharz enthält, das die Verdünnung in Wasser erleichtert.

## Claims

1. Electroapplicable cationic paint composition containing the product obtained from the reaction of
(a) an amine-diol obtained from the reaction of a primary amine having the formula R-NH₂, in which R represents an aliphatic or aromatic radical, with at least propylene oxide or a mixture of alkylene oxides comprising predominantly propylene oxide, and
(b) at least one polyfunctional substance, capable of acting on the hydroxyl groups of said amine-diol.

2. Composition as claimed in claim 1, characterized in that the R group of the amine R-NH₂ contains from 1 to 20 carbon atoms.

3. Composition as claimed in one of claims 1 and 2, characterized in that the R group is an alkyl or aralkyl group with a linear or branched chain.

4. Composition as claimed in one of claims 1 to 3 characterized in that the R group contains from 1 to 12 carbon atoms.

5. Composition as claimed in one of claims 1 to 4, characterized in that the R group is selected from the methyl, butyl or lauryl radicals.

6. Composition as claimed in one of claims 1 to 4, characterized in that the R group is of an aromatic nature, in which case it may contain from 6 to 18 carbon atoms, and preferably from 6 to 12 carbon atoms.

7. Composition as claimed in claim 6, characterized in that R is a phenyl group.

8. Composition as claimed in one of claims 1 to 7, characterized in that the propylene oxide is present in proportions higher than 50% by weight , and preferably higher than 80% by weight with respect to the mixture of alkylene oxides.

9. Composition as claimed in one of claims 1 to 8, characterized in that the amine-diol (a) has a molecular weight of between approximately 800 and 1500, and preferably between 1000 and 1200.

10. Composition as claimed in one of claims 1 to 9, characterized in that said polyfunctional substance (b) is selected from isocyanates, acids and acid anhydrides.

11. Composition as claimed in claim 10, characterized in that the isocyanates are selected from diisocyanates, such as toluene diisocyanate, isophorone diisocyanate, hexamethylene diisocyanate, and tetramethylphenyl isocyanate.

12. Composition as claimed in claim 10, characterized in that the diacids or diacid anhydrides are phthalic and maleic acids or anhydrides.

13. Composition as claimed in one of claims 1 to 11, characterized in that , for the reaction of the amine-diol (a) with the polyfunctional substance (b), the ratio OH/NCO expressed in molar equivalents, of the hydroxyl groups of amine-diol (a) to the isocyanate groups of substance (b) ranges approximately between 2/1 and 4/3.

14. Composition as claimed in claim 13, characterized in that the ratio OH/NCO is approximately 3/2.

15. Composition as claimed in one of claims 1 to 10 and 12, characterized in that for the reaction of the amine-diol (a) with the polyfunctional substance (b), the ratio OH/CO₂H expressed in molar equivalents of the hydroxyl groups of the amine-diol (a) to the acid or anhydric groups of substance (b) ranges approximately between 2/1 and 4/3, and is preferably approximately 3/2.

16. Composition as claimed in one of claims 1 to 15, characterized in that it also contains a milling resin to facilitate dilution in water.
